# EUROPÄISCHE PATENTANMELDUNG

(11) **EP 2 742 914 A1**
(43) Veröffentlichungstag der Anmeldung: **18.06.2014**
(21) Anmeldenummer: 12197503.1
(22) Anmeldetag: 17.12.2012
(51) Int. Cl.: A61F 2/44, A61B 17/70

(54) **Stufenlos höhenverstellbares Zwischenwirbelfusionsimplantat**

(71) Anmelder: FACET-LINK Inc., Rockaway NJ 07866 (US)
(72) Erfinder: Dmuschewsky, Klaus, 22397 Hamburg (DE)
(74) Vertreter: Glawe, Delfs, Moll

(57) **Zusammenfassung**

Die Anmeldung betrifft ein Zwischenwirbelfusionsimplantat zur Fusion zweier benachbarter Wirbel umfassend ein Bodenstück (2) und ein Deckstück (3) sowie eine Spreizeinrichtung, wobei das Boden- und das Deckstück (2, 3) durch eine zwischen ihnen liegende außen angelenkte Wippe (4) verbunden sind, sowie die Spreizeinrichtung eine Spreizklammer (6, 6') an der Wippe ist und eine Schubeinrichtung (5) für die Spreizklammer vorgesehen ist. Mittels der Spreizklammer wird die Wippe aufgerichtet, so dass das Implantat höhenmäßig expandiert. Dank der äußeren Anlenkung der Wippe steht ein großer Hubweg zur Verfügung. Weiter wirkt die Spreizklammer durch ihre Reibkräfte hemmend gegenüber einer unerwünschten Rückstellung.

## Beschreibung

Die Erfindung betrifft ein Implantat zur Fusion zweier benachbarter Wirbel umfassend ein Bodenstück und ein Deckstück sowie eine Spreizeinrichtung.

Durch Verschleiß oder krankhafte Veränderungen kommt es an der Wirbelsäule zu einer Degeneration von Bandscheiben. Soweit konservative Therapie mit Medikation und/oder Physiotherapie nicht greift, ist mitunter eine operative Behandlung indiziert. Dazu ist es bekannt, in den Wirbelzwischenraum mit der degenerierten Bandscheibe ein bewegliches oder unbewegliches Implantat einzuführen. Dieses übernimmt die Tragfunktion der degenerierten Bandscheibe und stellt insoweit wieder eine sichere Abstützung zwischen den benachbarten Wirbeln her. Unbewegliche Implantate werden auch als "Fusionsimplantat" bezeichnet.

Zur Implantation der Fusionsimplantate sind verschiedene Operationstechniken bekannt. Eine klassische Operationstechnik besteht in einem Zugang von ventral, um so die Gefahr einer Beschädigung des Rückenmarks in der Wirbelsäule zu vermeiden. Dieser Vorteil wird jedoch erkauft mit einem ausgesprochen langen Zugangsweg durch den Bauch- oder Brustraum des Patienten. Da es hierbei zu Komplikationen kommen kann, ist ein alternativer Zugangsweg etabliert worden, nämlich von dorsal. Dieser bietet zwar den Vorteil eines kurzen Wegs, jedoch besteht die Gefahr einer Kollision bzw. Verletzung des Rückenmarks. Um diese Gefahr klein zu halten, erfolgt die Operation üblicherweise im Weg minimal invasiver Chirurgie. Derartige Zugänge von unmittelbar dorsal bzw. mehr von der Seite sind als Operationstechnik PLIF (posterior lumbar intervertebral fusion) bzw. TLIF (transforaminelle interkorporelle lumbare Fusion) bekannt, bei der die Bandscheibe von posterior bzw. lateral exponiert wird. Wegen der kleinen Querschnitte beim Zugang mittels minimal invasiver Chirurgie ist hierbei die Größe der Fusionsimplantate naturgemäß stark beschränkt.

Für die Therapie mittels der PLIF- bzw. TLIF-Technik sind sehr kleine Fusionsimplantate bekannt. Sie bieten den Vorteil, dass sie durch die minimal invasive Chirurgie dank ihrer Kleinheit implantiert werden können. Ein inhärenter Nachteil ihrer Kleinheit liegt jedoch darin, dass die Stützfunktion aufgrund der geringen Abmessungen eingeschränkt und mitunter unzureichend ist. Eine größere Ausführung der Fusionsimplantate würde die Stützfunktion zwar verbessern, jedoch ist dies nicht praktikabel aufgrund der Beschränkung der minimal invasiven Chirurgie.

Die Erfindung hat sich zur Aufgabe gesetzt, ein Fusionsimplantat der eingangs genannten Art dahingehend zu verbessern, dass bei weiterhin kleinem Zugangsquerschnitt, wie er für die minimal invasive Chirurgie üblich ist, dennoch auch bei großen Wirbelzwischenräumen eine bessere Stützwirkung erzielen kann.

Die erfindungsgemäße Lösung liegt in den Merkmalen des unabhängigen Anspruchs. Vorteilhafte Weiterbildungen sind Gegenstand der abhängigen Ansprüche.

Bei einem Zwischenwirbelfusionsimplantat zur Fusion zweier benachbarter Wirbel umfassend ein Bodenstück und ein Deckstück sowie eine Spreizeinrichtung ist erfindungsgemäß vorgesehen, dass das Boden- und das Deckstück durch eine zwischen ihnen liegende außen angelenkte Wippe verbunden sind, wobei die Spreizeinrichtung eine Spreizklammer an der Wippe ist und eine Schubeinrichtung für die Spreizklammer vorgesehen ist.

Die Erfindung beruht auf dem Gedanken, mittels der Schubeinrichtung die außen angelenkte Wippe aufzurichten, wobei die Spreizklammer hier als Übertragungsglied wirkt. Durch das Aufrichten der Wippe entfernt sich das Deckstück von dem Bodenstück, so dass das Zwischenwirbelfusionsimplantat höhenmäßig expandiert. Dank der äußeren Anlenkung der Wippe steht hiermit ein verhältnismäßig großer Hubweg zur Verfügung. Außer dem verhältnismäßig großen Hubweg bietet die Übertragung mittels der Spreizklammer auf die Wippe den Vorteil, dass durch die Verwendung der Spreizklammer als Übertragungsglied mehr Reibstellen zur Verfügung stehen, welche eine Hemmung bewirken. Damit wird der Gefahr einer unbeabsichtigten Rückstellung, d. h. dass sich die Höhe verringert, entgegengewirkt.

Das erfindungsgemäß ausgeführte Zwischenwirbelfusionsimplantat bietet weiter den Vorteil, dass es stufenlos verstellbar ist. Es kann damit besonders fein an die jeweiligen anatomischen Verhältnisse des Patienten angepasst werden. Ein weiterer Vorteil besteht darin, dass es grundsätzlich rückstellfähig ist. Das heißt, wird die Schubeinrichtung in Gegenrichtung betätigt, so wird die Expansion zurückgenommen und die Höhe des Implantats verringert sich. Dies ist insbesondere für Testzwecke, aber auch zur Implantation an anatomisch schwierigen Stellen mitunter ein beträchtlicher Vorteil.

Zweckmäßigerweise sind zwei Spreizklammern gegenüberliegend vorgesehen. Dies bietet den Vorteil einer größeren sowie einer symmetrischen Kraftaufbringung von beiden Seiten. Durch die beidseitige Kraftaufbringung wird der Gefahr eines Verkippens zwischen Bodenstück und Deckstück bei der Hubbewegung entgegengewirkt.

Eine zweckmäßige Ausführung der Schubeinrichtung ist als ein Kontraktionselement. Hierbei wird die Spreizklammer gegen ein Gegenlager, welches auch durch eine zweite Spreizklammer gebildet sein kann, gezogen und spreizt sich dabei auf. Dies ermöglicht eine mechanisch besonders günstige lineare Ausführung, welche unter Bauraumgesichtspunkten erhebliche Vorteile in Bezug auf ihre Kompaktheit hat. Eine besonders zweckmäßige Ausführung ist die Verwendung einer Schraubenspindel als Stellglied für die Schubeinrichtung. Sie ermöglicht eine hohe Kraftübersetzung bei gleichzeitig geringen Bauraumanforderungen. Weiter bietet die Schraubenspindel den Vorteil einer inhärenten Selbsthemmung, wodurch es nicht zum unbeabsichtigten Rücklauf der erreichten Expansion kommen kann. Andererseits ermöglicht es die Schraubspindel aber, durch eine Betätigung in der Gegenrichtung die Expansion planmäßig zurückzunehmen.

Vorzugsweise ist die Wippe integral mit ihren Gelenken ausgeführt. Dies verringert die Teilezahl und vereinfacht die Herstellung. Durch die integrale Herstellung ist auch sichergestellt, dass stets zueinander passende Teile verwendet werden, so dass es anders als bei gesonderten Teilen nicht zu Fehlpassungen aufgrund ungünstiger Toleranzen kommen kann. Besonders zweckmäßig ist es hierbei, wenn die Wippe einstückig mit dem Bodenstück und/oder Deckelstück ausgeführt ist.

Zweckmäßig ist eine Ausführung mit Doppelwippe. Dies vergrößert den Stellbereich, genauer gesagt die maximale Expansion beträchtlich bei nur geringem Zusatzaufwand.

Zur Betätigung der Schubeinrichtung ist zweckmäßigerweise an einer posterioren Stirnseite ein Betätigungsanschluss vorgesehen. Diese Anordnung an der Stirnseite bietet den Vorteil, dass der Betätigungsanschluss durch denselben minimal invasiven Zugang erreicht werden kann, durch welchen auch das erfindungsgemäße Zwischenwirbelfusionsimplantat eingesetzt wird. Er ist damit gut zugänglich auch für den Fall einer Re-Operation. Besonders zweckmäßig ist die Ausführung des Betätigungsanschlusses als eine Schraubkupplung. Hierbei kann es sich insbesondere um eine Mutter handeln, welche an der Betätigungsspindel befestigt ist.

Vorzugsweise ist in der Deckfläche und/oder Bodenfläche mindestens eine Durchgangsöffnung ausgebildet. Diese ermöglicht es, dass Knochenmaterial besser in den Innenbereich des Implantats einwachsen kann. Damit wird die angestrebte Fusion der beiden benachbarten Wirbelkörper beschleunigt.

Die Erfindung wird nachfolgend unter Bezugnahme auf die beigefügte Zeichnung anhand zweier Ausführungsbeispiele näher erläutert. Es zeigen:
- Fig. 1:: eine schematische Ansicht für ein Zwischenwirbelfusionsimplantat gemäß einem ersten Ausführungsbeispiel im implantierten Zustand an Wirbelkörpern;
- Fig. 2:: eine perspektivische Ansicht des ersten Ausführungsbeispiels in einer Montagestellung;
- Fig. 3:: eine perspektivische Ansicht des ersten Ausführungsbeispiels in seiner Wirkstellung im expandierten Zustand;
- Fig. 4:: eine perspektivische Ansicht eines zweiten Ausführungsbeispiels in einer Montagestellung; und
- Fig. 5:: eine perspektivische Ansicht des zweiten Ausführungsbeispiels in seiner Wirkstellung im expandierten Zustand.

Ein in seiner Gesamtheit mit der Bezugsziffer 1 bezeichnetes Zwischenwirbelfusionsimplantat ist zur Implantation in einem Wirbelzwischenraum 91 zwischen zwei unmittelbar benachbarten Wirbelkörpern 9, 9' vorgesehen. Bei physiologisch intakter Wirbelsäule befindet sich zwischen den Wirbeln im Wirbelzwischenraum eine Bandscheibe 90. Sie kann aufgrund von Krankheit oder Verschleiß degeneriert sein, so dass sie zumindest teilweise reseziert werden muss. Um trotz des Verlustes an Bandscheibenmaterial eine ausreichende Abstützung des Wirbelzwischenraums 91 zu erreichen und somit ein Kollabieren der Wirbelsäule zu verhindern, ist das Zwischenwirbelfusionsimplantat 1 in den Wirbelzwischenraum 91 eingesetzt. Es wirkt stützend und erleichtert somit eine Fusion der beiden benachbarten Wirbel 9, 9' auf natürlichem Wege durch Knochenwuchs.

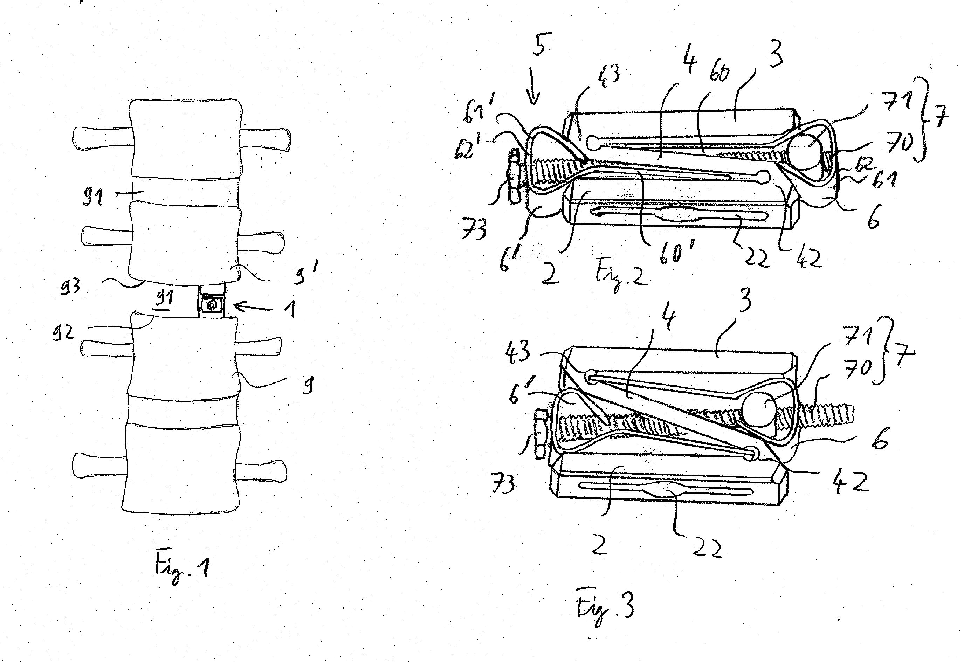

Ein in den Figuren 2 und 3 dargestelltes erstes Ausführungsbeispiel für ein erfindungsgemäßes Zwischenwirbelfusionsimplantat, welches in seiner Gesamtheit mit der Bezugsziffer 1 bezeichnet ist, umfasst ein Bodenstück 2 sowie ein Deckstück 3 mit einer dazwischen liegenden Wippe 4. Die Wippe 4 ist hierbei über zwei Gelenke 42, 43 an ihren äußeren Enden einstückig mit dem Bodenstück 2 bzw. dem Deckstück 3 verbunden. Die Gelenke 42 und 43 sind an den gegenüberliegenden Enden der Wippe 4 und auch an den gegenüberliegenden Seiten des Bodenstücks 2 und des Deckstücks 3 angeordnet. Im dargestellten Ausführungsbeispiel befindet sich das die Wippe 4 mit dem Bodenstück 2 verbindende Gelenk 42 am anterioren Ende (in der Darstellung in Fig. 2 und 3 auf der rechten Seite), während sich das die Wippe 4 mit dem Deckstück 3 verbindende Gelenk 43 am posterioren Ende befindet. Insgesamt ergibt sich damit eine Art Z-Form für das erste Ausführungsbeispiel.

An dem Bodenstück 2 ist eine Durchbrechung 22 angeordnet. Sie verbindet den Raum unterhalb des Bodenstücks 2 mit dem Zwischenraum zwischen dem Bodenstück 2 und der Wippe 4 bzw. dem Deckstück 3.

In den nach anterior offenen Zwischenraum zwischen der Wippe 4 und dem Deckstück 3 ist eine Spreizklammer 6 eingeschoben. Sie weist ein Führstück 60 auf, welches sich zungenartig in den Zwischenraum zwischen Wippe 4 und Deckstück 3 einschiebt. Mit seinem freien Ende ist es in der Montagestellung, wie in Fig. 2 dargestellt, beabstandet von dem Gelenk 43 zwischen dem Deckstück 3 und der Wippe 4. An seinem anderen Ende setzt sich das Führstück 60 einstückig fort in einer Halteschlaufe 61. Die Halteschlaufe weist eine sich nach oben verengende U-Form auf, wobei der eine Schenkel des U an dem Deckstück 3 und der andere Schenkel des U an der Wippe 4 im Bereich des Gelenks 42 anliegt. Die beiden Schenkel sind verbunden durch ein Basisstück der U-förmigen Schlaufe 61, welche das anteriore Ende der Spreizklammer 6 bildet. Dort ist eine Öffnung 62 für den Durchgang einer Schraubspindel 70 vorgesehen. Die Schraubspindel 70 erstreckt sich hierbei von dem posterioren Ende durch die Wippe 4 hindurch parallel zum Führstück 60 durch den Innenraum der U-förmigen Halteschlaufe 61 und endet in der Montagestellung im Bereich der Öffnung 62.

Die Größe der Schlaufe 61 ist so bemessen, dass in ihr ein Gegenlager 71 für die Schraubspindel 70 aufgenommen ist. Das Gegenlager 71 ist in dem dargestellten Ausführungsbeispiel als ein zylindrischer Körper gebildet, der quer zu seiner Zylinderachse eine Durchgangsbohrung mit einem zu der Schraubspindel 70 komplementären Innengewinde aufweist.

In dem dargestellten Ausführungsbeispiel ist eine zweite Spreizklammer 6' vorgesehen, welche in komplementärer Weise in den nach posterior öffnenden Zwischenraum zwischen Bodenstück 2 und Wippe 4 eingeschoben ist. Es ist gleichartig aufgebaut mit einem Führstück 60' und einer U-förmigen Schlaufe 61' mit einer stirnseitigen Öffnung 62'. Durch diese ist das andere Ende der Schraubspindel 70 gesteckt, wobei an den sich außerhalb der U-förmigen Schlaufe 61' befindenden posterioren Enden der Schraubspindel 70 eine Schraubkupplung 73 in Gestalt einer angeschweißten Mutter befindet.

Das erfindungsgemäße Zwischenwirbelfusionsimplantat wird in der dargestellten Montagestellung (siehe Fig. 2) durch einen minimal invasiven Zugang an seinen Bestimmungsort im Zwischenwirbelraum 91 eingesetzt. Nach dem Einsetzen wird es expandiert, um so zu seinem Deckstück 2 an der oberen Deckplatte 92 des unteren benachbarten Wirbels und mit seinem Deckstück 3 in Anlage an die untere Deckplatte 93 des oberen benachbarten Wirbels 9' zu gelangen.

Die Betätigung des Zwischenwirbelfusionsimplantats zur Expansion geschieht dabei auf folgende Weise: An die Mutter als Schraubkupplungsanschluss 73 wird ein entsprechend passender Schraubenschlüssel angesetzt. Durch Verdrehen der Mutter mit der daran verschweißten Schraubspindel 70 kontrahiert eine aus der Schraubspindel 70 und dem Gegenlager 71 sowie der Mutter 73 gebildete Kontraktionseinrichtung, so dass sich die Spreizklammer 6 in den Zwischenraum zwischen Wippe 4 und Deckstück 3 hineinzieht. Für die gegenüberliegend angeordnete Spreizklammer 6' gilt das Entsprechende. Durch das Hineinziehen der Spreizklammern 6, 6' in die jeweiligen Zwischenräume kommen die U-förmigen Schlaufen 61, 61' mit ihren zulaufenden Schenkeln in Anlage an die äußeren Enden des Deckstücks 3 bzw. des Gelenks 42 für die Spreizklammer 6 und das Bodenstück 2 und das Gelenk 43 für die Spreizklammer 6', wodurch der jeweilige Zwischenraum zwischen der Wippe und dem Bodenstück 2 bzw. dem Deckstück 3 aufgeweitet wird.

Damit expandiert das Implantat in der Höhe, d. h. der Abstand zwischen dem Bodenstück 2 und dem Deckstück 3 vergrößert sich. Ein derartiger Expansionszustand, wie er für den Verbleib des Implantats vorgesehen ist, ist in Fig. 3 dargestellt. Er ist als Wirkstellung bezeichnet. Man erkennt, dass die durch die Schubeinrichtung 7 nach innen gezogenen Spreizklammern 6, 6' die Zwischenräume aufweiten und damit der Abstand zwischen dem Bodenstück 2 und dem Deckstück 3 vergrößert ist.

Es sei angemerkt, dass diese Expansion stufenlos erfolgt und auch jederzeit durch Rückdrehen der Schraubspindel 70 ganz oder teilweise reversiert werden kann. Weiter sei angemerkt, dass durch die gewährte Anordnung von Schraubspindel 70 mit dem Gegenlager 71, welches eine Durchgangsbohrung mit einem Innengewinde aufweist, eine Selbsthemmung bewirkt ist. Der Abstand kann sich damit nicht verringern, ohne ausdrückliche entsprechende Betätigung seitens des Chirurgen.

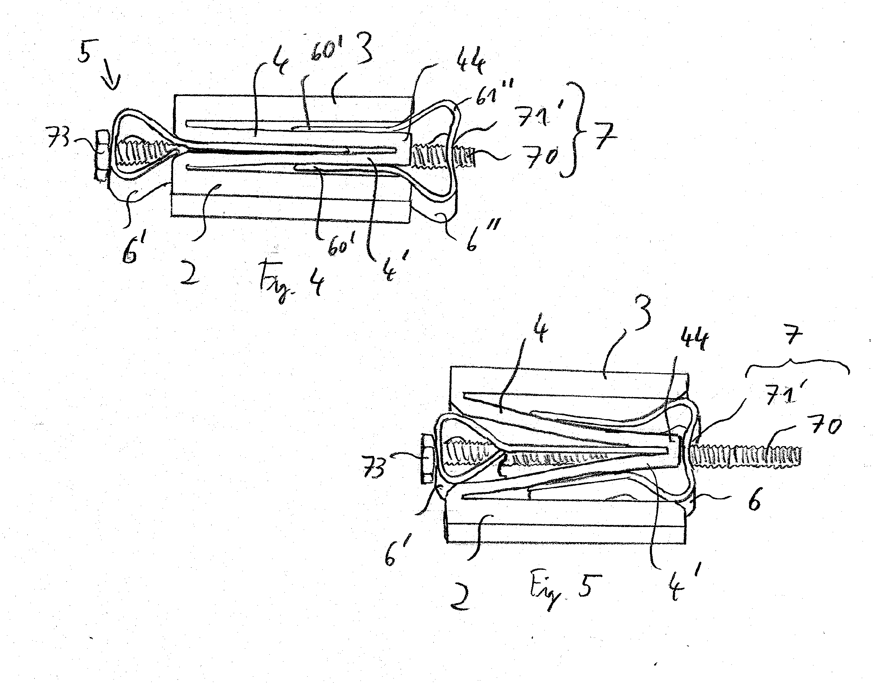

In den Figuren 4 und 5 ist ein zweites Ausführungsbeispiel dargestellt. Gleichartige Teile sind mit gleichen Bezugsziffern versehen wie bei dem ersten Ausführungsbeispiel.

Es unterscheidet sich von dem ersten Ausführungsbeispiel im Wesentlichen dadurch, dass die Wippe als eine Doppelwippe 4, 4' ausgeführt ist mit einem zusätzlichen Gelenk 44 zwischen den beiden Wippen 4, 4'. Damit erweitert sich die Z-Form des ersten Ausführungsbeispiels zu einer Art M-Form. Zur anterioren Seite gibt es damit zwei Zwischenräume, nämlich die zwischen dem Deckstück 3 und der Wippe 4 einerseits, wie auch bei dem ersten Ausführungsbeispiel, und nun auch der zwischen dem Bodenstück 2 und der zusätzlichen Wippe 4'. Die entsprechende anteriore Spreizklammer 6 ist daher so modifiziert, dass sie nun zwei Führstücke 60, 60' aufweist, wobei das zusätzliche Führstück 60' in den neu geschaffenen nach anterior offenen Zwischenraum zwischen dem Bodenstück 2 und der zusätzlichen Wippe 4' eingreift.

Als Gegenlager fungiert ein Innengewinde 71', welches in einer Öffnung am Basisteil der U-förmigen Schlaufe 61" angeordnet ist. Im Übrigen entspricht die Funktionsweise derjenigen des ersten Ausführungsbeispiels. Beim Betätigen der Schubeinrichtung an dem Schraubenkopf 73 werden die beiden Spreizklammern 6'', 6' in die Zwischenräume gezogen, wodurch die Zwischenräume aufgespreizt werden und sich das Deckstück 3 von dem Bodenstück 2 entfernt.

## Patentansprüche

1. Zwischenwirbelfusionsimplantat zur Fusion zweier benachbarter Wirbel umfassend ein Bodenstück (2) und ein Deckstück (3) sowie eine Spreizeinrichtung,
**dadurch gekennzeichnet, dass**
das Boden- und das Deckstück (2, 3) durch eine zwischen ihnen liegende außen angelenkte Wippe (4) verbunden sind, wobei die Spreizeinrichtung eine Spreizklammer (6) an der Wippe ist und eine Schubeinrichtung (5) für die Spreizklammer (6) vorgesehen ist.

2. Zwischenwirbelfusionsimplantat nach Anspruch 1, **dadurch gekennzeichnet, dass** mindestens zwei Spreizklammern (6, 6') einander gegenüberliegend vorgesehen sind.

3. Zwischenwirbelfusionsimplantat nach Anspruch 1 oder 2, **dadurch gekennzeichnet, dass** die Schubeinrichtung (5) als ein Kontraktionselement ausgeführt ist.

4. Zwischenwirbelfusionsimplantat nach einem der vorangehenden Ansprüche, **dadurch gekennzeichnet, dass** eine Schraubspindel (70) ein Stellglied für die Schubeinrichtung (5) bildet.

5. Zwischenwirbelfusionsimplantat nach einem der vorangehenden Ansprüche, **dadurch gekennzeichnet, dass** die Wippe (4) integral ausgeführte Gelenke (42, 43) aufweist.

6. Zwischenwirbelfusionsimplantat nach Anspruch 5, **dadurch gekennzeichnet, dass** die Wippe (4) einstückig mit dem Boden- und/oder Deckstück (2, 3) ausgeführt ist.

7. Zwischenwirbelfusionsimplantat nach einem der vorangehenden Ansprüche, **dadurch gekennzeichnet, dass** die Wippe (4) als eine Doppelwippe (4') ausgeführt ist.

8. Zwischenwirbelfusionsimplantat nach einem der vorangehenden Ansprüche, **dadurch gekennzeichnet, dass** an einer posterioren Stirnseite ein Betätigungsanschluss für die Schubeinrichtung angeordnet ist.

9. Zwischenwirbelfusionsimplantat nach Anspruch 8, **dadurch gekennzeichnet, dass** der Betätigungsanschluss als eine Schraubkupplung (73) ausgeführt ist.

10. Zwischenwirbelfusionsimplantat nach einem der vorangehenden Ansprüche, **dadurch gekennzeichnet, dass** an seinem Deckstück (2) und/oder Bodenstück (3) mindestens eine Durchgangsöffnung (22) ausgebildet ist.
